# EUROPEAN PATENT APPLICATION

(11) **EP 0 401 603 A1**
(43) Date of publication of application: **12.12.1990**
(21) Application number: 90109851.7
(22) Date of filing: 23.05.1990
(51) Int. Cl.: C07C 317/14

(54) **1,4"-(Bishalophenylsulfone) terphenyl**

(30) Priority: 09.06.1989 US 364728
(71) Applicant: GENERAL ELECTRIC COMPANY, Schenectady New York 12305 (US)
(72) Inventor: Peters, Edward Norman, Lenox, Massachusetts 01240 (US)
(74) Representative: Pratt, Richard Wilson

(57) **Abstract**

1,4˝-(bishalophenylsulfone)terphenyl and polymers therefrom are provided. The polymers exhibit high heat distortion temperatures, and high levels of chemical resistance and thermo-oxidative stability.

## Description

### BACKGROUND OF THE INVENTION

### Field of the Invention

This invention relates to monomers for the production of polyarylsulfones and, more particularly, relates to 1,4˝-bis(p-chlorophenylsulfone) terphenyl.

### Description of Related Art

Various monomers are known for the production of poly(aryl ether sulfones), see e.g. POLYMER, 1974, Vol 15, July, pages 456-465. Typically the monomers produce poly(aryl ether sulfones) which are amorphous materials and although a few of these poly(aryl ether sulfones) can be induced to crystallize by solvent treatment, few, if any, have been found which will crystallize from the melt. Consequently, the softening behavior of these resultant polymers is determined by their glass transition temperatures as is the case with primarily amorphous materials. The softening behavior of crystalline polymers, in contrast, especially if reinforced, is dependent on the melt temperature of the polymer rather than upon the glass transition temperature thereof. Thus, crystalline polymers, as a general rule, offer acceptable levels of performance at much higher temperatures than do amorphous polymers having somewhat similar polymeric structures. While crystallinity may be induced in a few known poly(aryl ether sulfones) by the employ of solvents, such solvent induced crystallization has a number of problems associated with it, including additional processing, significant reductions in important mechanical properties, and added material cost.

Accordingly, one object of the present invention is to provide a monomer for the production of a poly(aryl ether sulfone) which will crystallize from the melt.

Another object is to provide a monomer for the production of melt crystallizable poly(aryl ether sulfones) having both excellent chemical resistance and thermo-oxidative stability.

Another object is to provide a monomer for the production of a poly(aryl ether sulfone) having a high heat distortion temperature for use in high heat applications.

### SUMMARY OF THE INVENTION

The monomer of the present invention is 1,4˝-(bishalophenylsulfone)terphenyl which is derived from terphenyl and p-halobenzenesulfonyl halide using a catalyst. By reacting the 1,4˝-(bishalophenylsulfone)terphenyl with a dihydric phenol a poly(aryl ether sulfone) is obtained which (a) is generally crystallizable from the melt, (b) exhibits high levels of chemical resistance and thermo-oxidative stability, and (c) has a high heat distortion temperature.

### DETAILED DESCRIPTION OF THE INVENTION

The present invention involves a monomer, 1,4˝-(bishalophenylsulfone)terphenyl useful in the production of poly(aryl ether sulfones).

The monomer, 1,4˝-(bishalophenylsulfonyl)terphenyl, is prepared from a reaction mixture of p-terphenyl and p-halobenzenesulfonyl halide using a metal halide catalyst. In general, the reaction appears as follows: 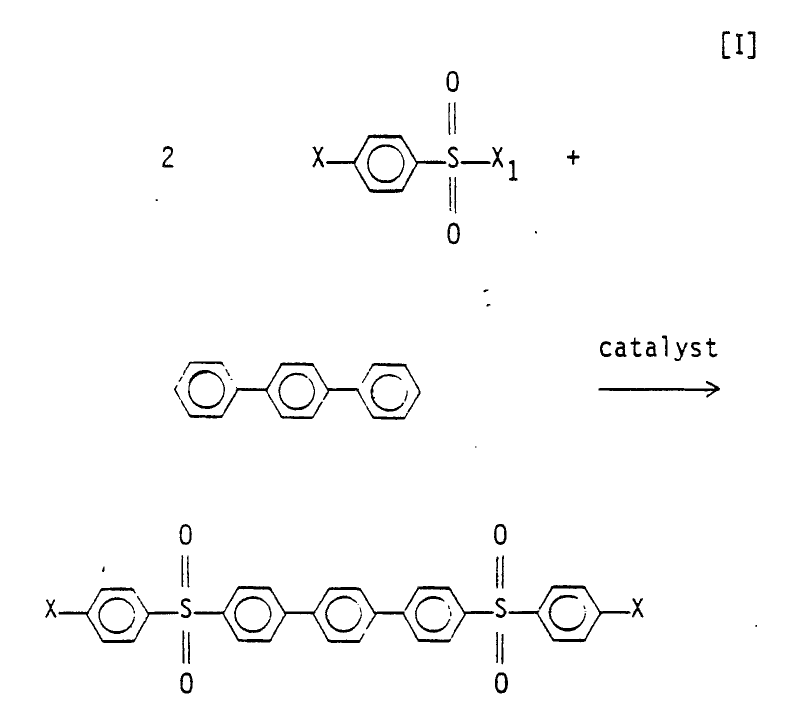 wherein X and X₁ are independently selected from halogens. Preferably X is selected from chlorine, bromine and fluorine. Preferably X₁ is selected from chlorine and bromine. More preferably, the monomer prepared is 1,4˝-bis(p-chlorophenylsulfone)terphenyl and is derived from a reaction mixture of p-terphenyl, p-chlorobenzenesulfonyl chloride in the presence of an organic solvent and a metal chloride catalyst, preferably ferric chloride. The reaction may be represented as follows:

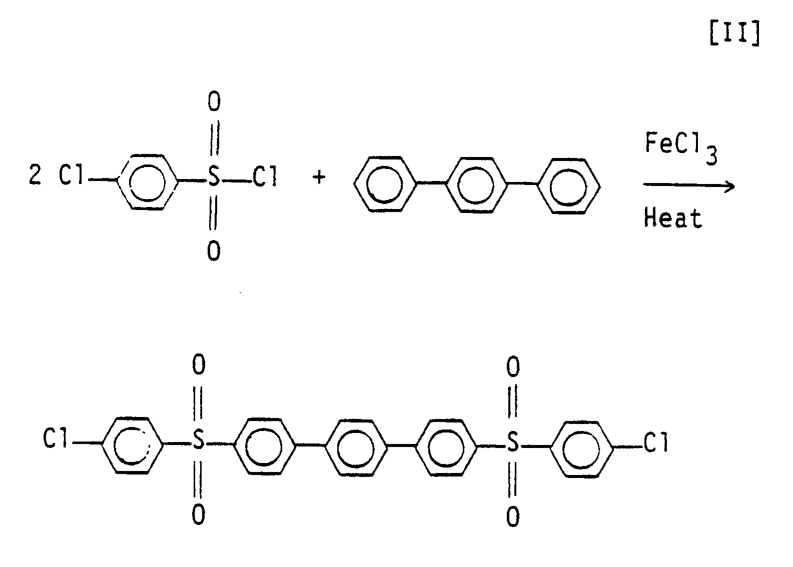

Preferably the reaction rate is promoted by heating the reaction mixture to a temperature selected from between 110°C and 170°C and more preferably between 150°C and 170°C for a period of at least about 0.5 hours to obtain a substantial yield of the monomer. The organic solvent of the reaction mixture is 1,2,4 trichlorobenzene although other organic solvents which are suitable for dissolving terphenyl and the p-halobenzenesulfonyl halide may also be employed. The amounts of p-halobenzenesulfonylhalide and terphenyl employed in the reaction mixture should be in mole ratios of about 2:1 and preferably a slight excess of p-halobenzenesulfonyl halide should be present, for example, a mole ratio of 2.1 to 1. After the reaction mixture is heated and allowed to react to form the 1,4˝-bis(p-halophenylsulfone)terphenyl monomer, the monomer can be isolated by filtration, washing and drying.

Under anhydrous conditions, poly(aryl ether sulfones) polymers may then be prepared in a dipolar aprotic solvent by reacting a dihydric phenol with the 1,4˝-bis(p-halophenylsulfone)terphenyl monomer in the presence of a base. Suitable anhydrous conditions can be obtained by the employ of a base such as sodium hydroxide (NaOH). The reaction can be represented as follows: 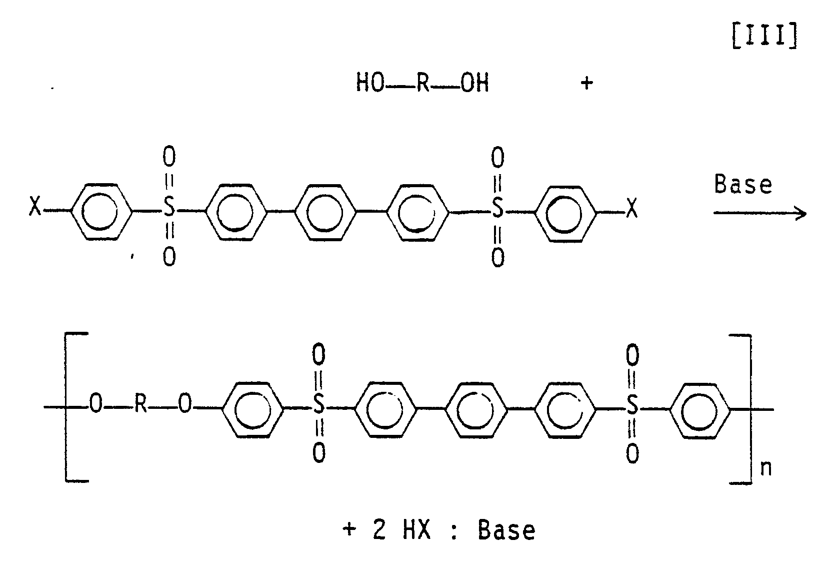 where R is a member of the class consisting of (i) 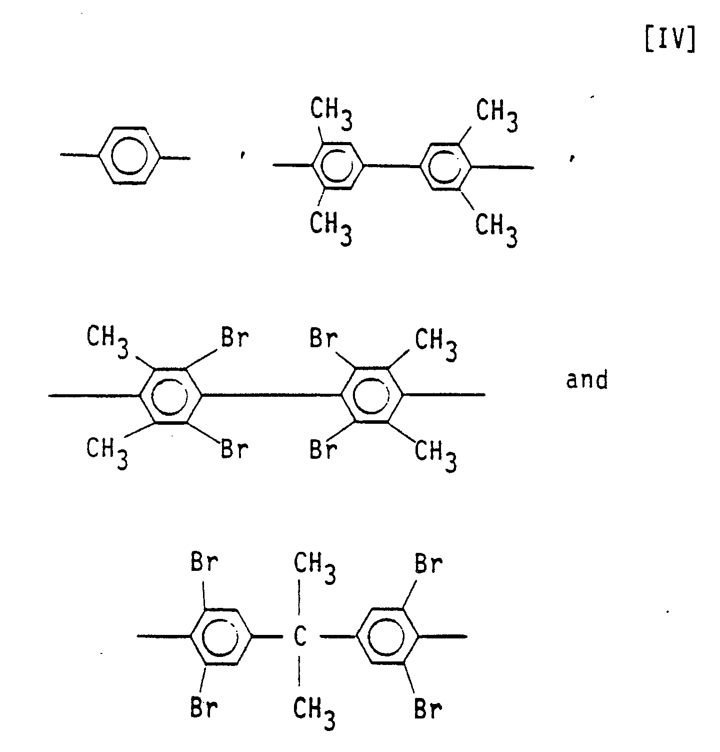 and (ii) divalent radicals of the general formula: 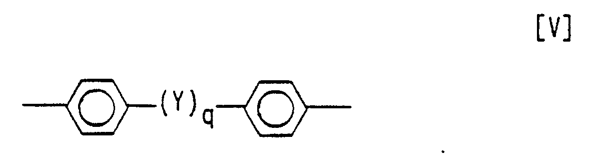 where Y is a member selected from the class consisting of divalent radicals of the formulas: 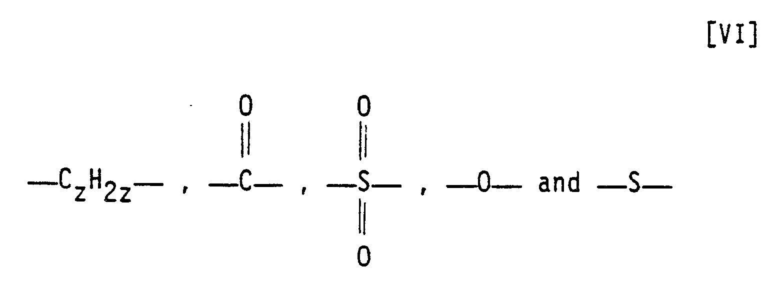 where q is 0 or 1, and z is a whole number from 1 to 5.

Suitable dihydric phenols include the dihydric phenols such as, for example, 2,2-bis(4-hydroxyphenyl)propane, bis(4-hydroxyphenyl)methane, 2,2-bis(4-hydroxy-3-methylphenyl)propane, 4,4-bis(4-hydroxyphenyl)-heptane, 2,2-(3,5,3′,5′-tetrachloro4,4′-dihydroxy- phenyl)propane, 2,2-(3,5,3′,5′-tetrabromo-4,4′- dihydroxyphenyl)methane. Other dihydric phenols which are also suitable for use in the preparation of the above polymers are disclosed in U.S. Patent Nos. 2,999,835; 3,038,365; 3,334,154; and 4,131,575; incorporated herein by reference.

The preferred dihydric phenols are bisphenol-A

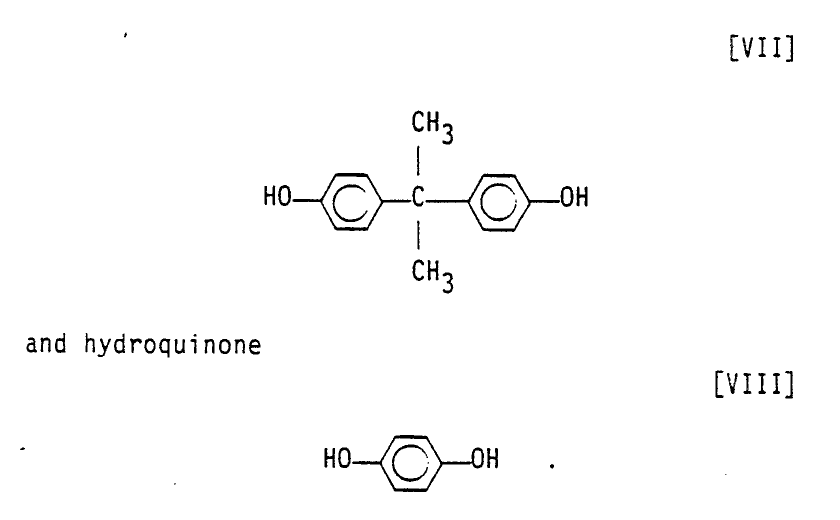

Thus, when bisphenol-A is the dihydric phenol, R is 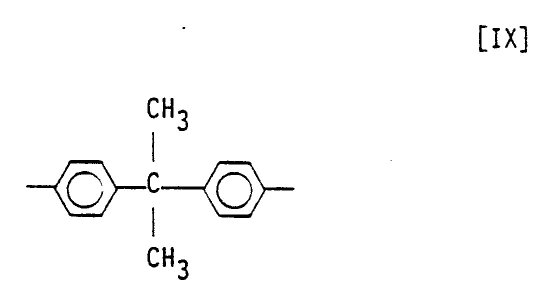 and when hydroquinone is the dihydric phenol, R is

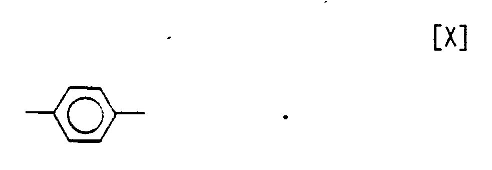

Preferably blends of dihydric phenols are not employed in that such blends generally reduce the tendencies of the polymer to crystallize from the melt.

The polymer may be produced by either a DMSO process or a DMAC process, similar suitable polymer forming processes are set forth in Johnson. et. al., U.S. Patent No. 4,175,175, which is incorporated herein by reference. The DMSO process involves the addition of a stoichometric amount of a base, preferably a metal hydroxide, e.g. NaOH, at 2 mole equivalents to the dihydric phenol in a dipolar aprotic solvent to form an anhydrous disodium salt of the dihydric phenol. The 1,4˝-bis(p-halophenylsulfone)terphenyl is then added to the solution containing the anhydrous disodium salt and heated to form the polymer. In more detail, the DMSO process involves preparing the poly(aryl ether sulfones) involves converting the dihydric phenol to the alkali metal salt by reaction of the alkali metal with the dihydric phenol in the dipolar aprotic solvent. A suitable dipolar aprotic solvent is dimethylsulfoxide, although other solvents may also be suitable. The solvent preferably also contains amounts of a cosolvent such as toluene or chlorobenzene which are used to azeotropically remove the water of reaction.

Another suitable process for the preparation of the poly(aryl ether sulfone) is the DMAC process which involves preparing a mixture of the 1,4˝-bis(p-halophenylsulfone)terphenyl, dihydric phenol, an excess of a base (i.e. K₂CO₃ at greater than 2 mole equivalents thereof), a solvent comprising a dipolar aprotic solvent and a cosolvent to azeotropically remove the water of reaction, heating the mixture and simultaneously removing water therefrom to form a reacted mixture comprising polymer and a metal halide salt. Suitable dipolar aprotic solvents include dimethylacetamide, although other dipolar aprotic solvents such as diphenyl sulfone are suitable. The cosolvent employed is toluene, although other cosolvents, for instance, benzene, heptane, xylene, chlorobenzene, dichlorobenzene, and the like, are suitable. The cosolvent may be present at levels as high as 50 weight percent of the combined solvent and cosolvent present.

It is desirable to exclude oxygen from the reaction mixture to avoid side reactions between diatomic oxygen and the reactants, solvents or polymers. A nitrogen atmosphere was employed to ensure the exclusion of diatomic oxygen from the presence of the reaction mixture.

The reaction temperature is preferably above 110°C in order to keep the polymer in solution and for improved reaction rates, more preferably the reaction temperature is between 150°C and 170°C. The reaction temperature should be below 190°C if dimethylsulfoxide is employed in order to avoid thermal decomposition thereof.

Reaction temperatures may be above the normal boiling point of the solvents if pressures in excess of atmospheric pressure are employed. The amount of solvent employed may vary, preferably the reaction mixture comprises 50% to 85% by weight solvent.

Following polymer formation, the polymer may be recovered from the solvent by conventional processes such as precipitation of the polymer in a nonsolvent, such as methanol, followed by washing and drying of the polymer. Molecular weight of the polymer may be controlled by the addition of chain stoppers to the reaction mixture.

The polymers prepared from dihydric phenols and 1,4˝-bis(p-chlorophenylsulfone)terphenyl were rapidly crystallized from the melt and exhibited high heat distortion temperatures.

### EXAMPLES

The following examples are provided by way of illustration and not by way of limitation.

### EXAMPLE 1

The monomer, 1,4˝-bis(p-chlorophenylsulfone) terphenyl was prepared as follows:

575.8 grams (2.5 moles) of p-terphenyl (M.W. 230.31) was added to 1500 milliliters of 1,2,4-trichlorobenzene to form a slurry, this slurry was then swept with N₂ for about 30 minutes. The slurry was then heated to 70°C without the terphenyl completely dissolving. The slurry was then further heated to 110°C without the p-terphenyl completely dissolving. 1108.13 grams (97%) of p-chlorobenzenesulfonylchloride (5.25 moles) (M.W. 211.07) was dissolved in 700 milliliters (ml) of 1,2,4-trichlorobenzene and added to the terphenyl slurry and thereby formed a homogenous solution. 55 grams of ferric chloride (FeCl₃, M.W. 162.2, 0.25 moles) was added to the solution to, form a reaction mixture. The mixture was heated and maintained within a temperature range of 150°C to 170°C for 20 hours to obtain a reacted mixture. The monomer, 1,4˝-bis(p-chlorophenylsulfone)terphenyl was then isolated from the reacted mixture by filtration of the reacted mixture, washing the resultant filtered solid with methanol, slurrying the washed solids with 3 liters of water and 60 grams of citric acid, heating and then filtering this slurry, washing these filtered solids with methanol and hot toluene, slurrying the solids in hot xylene and filtering off the xylene, slurrying the resultant solids in acetone followed by filtering off the solids therefrom. These solids were then dried at 100°C under vacuum for 6 hours and then were further dried at 130°C under vacuum for over 12 hours to yield 1160 grams of 1,4˝-bis(p-chlorosulfone)terphenyl having a Tm by differential scanning calorimetry of 351°C, a heat of fusion of 25.5 cal/gm, and a molecular weight of 579.50 grams per mole.

### EXAMPLE 2

A poly(aryl ether sulfone) was prepared by reacting 1,4˝-bis(p-chlorophenylsulfone)terphenyl and bisphenol-A. Bisphenol-A, K₂CO₃, diphenylsulfone, toluene and 1,4˝-bis(p-chlorophenylsulfonyl)terphenyl were admixed to form a reaction mixture. The reaction mixture was heated to a reaction temperature of 170°C with simultaneous removal of water from the mixture to form polymer in a reacted mixture. The reacted mixture was poured into methanol and then filtered. The filtered solids were then added to hot H₂O to form a slurry, filtered, added to H₂O, filtered, added to acetone, filtered, added to acetone, filtered, added to hot H₂O, filtered, and then dried by vacuum in a vacuum oven at 175°C. The resultant polymer had an intrinsic viscosity of 0.52 dl/g as determined in phenol/1,1,2,2 tetrachloroethane (TCE) and had a Tm of 338°C as determined by differential scanning calorimetry. The polymer was rapidly crystallizable from the melt.

### EXAMPLE 3

A poly(aryl ether sulfone) was prepared by reacting 1,4˝-bis(p-chlorophenylsulfone)terphenyl with hydroquinone. Under a nitrogen atmosphere 15.9 grams of 1,4˝-bis(p-chlorophenylsulfone)terphenyl (0.20 moles, M.W. 579.50), 22.02 grams of hydroquinone (0.20 moles, M.W. 110.10), 45 grams of K₂CO₃ (M.W. 138.21), 350 grams of diphenylsulfone and 100 ml of toluene were mixed together and were heated while water was simultaneously being removed by distillation to form polymer in a reacted mixture. The reacted mixture was removed from the flask, poured into methanol. The solids were then filtered out, slurried in hot H₂O, filtered out, slurried in hot H₂O, filtered out, slurried in acetone, filtered out, slurried in acetone, filtered out, slurried in hot H₂O, filtered, dried in a vacuum oven at 175°C. The resultant polymer was insoluble in a phenol/TCE solvent and had a melting temperature of 411°C. The polymer was rapidly crystallized from the melt.

The monomers of the present invention are useful in the preparation of polymers which are useful in the production of molded articles, especially glass reinforced molded articles.

## Claims

1. A composition comprising a compound represented by the structural formula: 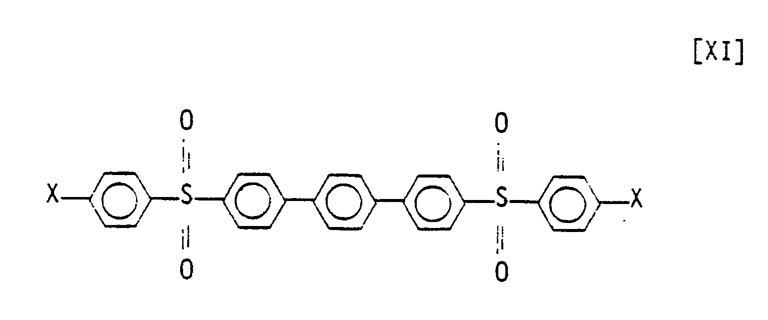 wherein X is independently selected from the group consisting of halogens.

2. The composition of Claim 1 wherein X and X₁ are chlorine.

3. A compound represented by the structural formula: 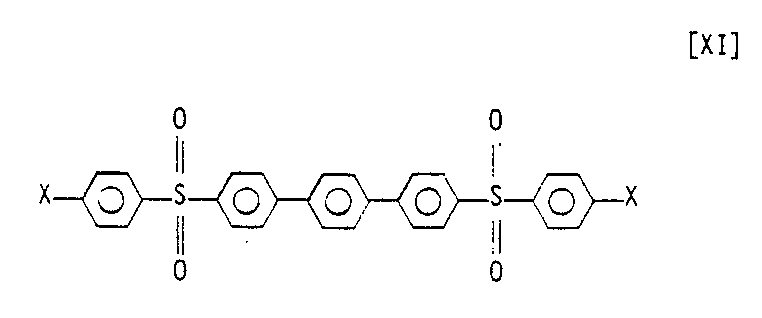 wherein X is independently selected from the group consisting of halogens.

4. The composition of Claim 3 wherein X and X₁ are chlorine.

5. A process for the production of 1,4˝-(bishalophenyl sulfone)terphenyl, said process comprising:
(a) admixing p-chlorobenzenesulfonyl chloride and terphenyl to form a reaction mixture;
(b) contacting said reaction mixture with a ferric chloride catalyst; and
(c) heating said reaction mixture to a temperature of from between 110°C and 170°C.
